(19) Europäisches Patentamt / European Patent Office / Office européen des brevets

(11) EP 3 075 852 B1

(12) # EUROPEAN PATENT SPECIFICATION

(45) Date of publication and mention of the grant of the patent: **26.12.2018 Bulletin 2018/52**

(51) Int Cl.: ***C12Q 1/6886*** *(2018.01)*

(21) Application number: **16163067.8**

(22) Date of filing: **31.03.2016**

(54) **METHOD FOR ACQUIRING INFORMATION ON GYNECOLOGIC CANCER AND GYNECOLOGIC CANCER DETECTION KIT**

VERFAHREN ZUR ERFASSUNG VON INFORMATIONEN ÜBER GYNÄKOLOGISCHEN KREBS UND NACHWEISKIT FÜR GYNÄKOLOGISCHEN KREBS

PROCÉDÉ D'ACQUISITION D'INFORMATIONS SUR UN CANCER GYNÉCOLOGIQUE ET KIT DE DÉTECTION D'UN CANCER GYNÉCOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2015 JP 2015073592**

(43) Date of publication of application: **05.10.2016 Bulletin 2016/40**

(73) Proprietors:
- **SYSMEX CORPORATION**
  **Kobe-shi,**
  **Hyogo 651-0073 (JP)**
- **The University of Tokyo**
  **Bunkyo-ku,**
  **Tokyo 113-8654 (JP)**

(72) Inventors:
- **Tai, Kaya**
  **Kobe-shi, Hyogo, 651-0073 (JP)**
- **Nagae, Genta**
  **Tokyo, 113-8654 (JP)**
- **Aburatani, Hiroyuki**
  **Tokyo, 113-8654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
- **Daniel Steinbach: "GENOMWEITE METHYLIERUNGSANALYSEN UND IDENTIFIZIERUNG PROGNOSTISCHER UND PRÄDIKTIVER MARKER FüR DAS Epitheliale Ovarialkarzinom", , 2 September 2014 (2014-09-02), XP055286607, Retrieved from the Internet: URL:http://d-nb.info/1064611060/34 [retrieved on 2016-07-07]**
- **DIANA L. KOLBE ET AL: "Differential Analysis of Ovarian and Endometrial Cancers Identifies a Methylator Phenotype", PLOS ONE, vol. 7, no. 3, 5 March 2012 (2012-03-05), page e32941, XP055267312, DOI: 10.1371/journal.pone.0032941**
- **XUE-LIAN DU ET AL: "Gene alterations in tumor-associated endothelial cells from endometrial cancer", INTERNATIONAL JOURNAL OF MOLECULAR MEDICINE, vol. 22, 1 January 2008 (2008-01-01), pages 619-632, XP055286700, GR ISSN: 1107-3756, DOI: 10.3892/ijmm_00000064**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> THE UNIVERSITY OF TOKYO
SYSMEX CORPORATION

<120> Method for obtaining information on gynecologic cancer and
gynecologic cancer detection kit

<130> SYSM-105-EP

<150> JP 2015-073592
<151> 2015-03-31

<160> 7

<170> PatentIn version 3.5

<210> 1
<211> 4123
<212> DNA
<213> Homo sapiens

<400> 1

```
gggcaattcc cagggaactg ggccatcagg aactgcactc agggcctcgt tctgtgtcta      60
ccttcctggg gttctgccat gcctagactc acagttcact cccagccagg cacctccaac     120
caacagtccc ccaacatacc acacagatga ggtagatacc caggaacacc tggccggtgg     180
actgcaggga gcggctgcga ggtttccggc ggtacagctc cccagcaccg ctggccaaca     240
caagaaagcc gccgatgatg gcaactgtgc gcgagtacat acggacctag gccggggtaa     300
ggggacccca gtcaagaact gcagccgcca catctctcct ggcttcccag agtgtagaac     360
cacccctctc tacacatccc attccatctg atgaaccctc ttacatttcc aagcttggtg     420
ggcaggtgca acagagacag atcattcttt ttatatatat atagaagtag aaactgaggc     480
ccagagggtt aagaaaactg tcaggtctgg ccaggcgcag tggctcacgc ctgtaatccc     540
agcactttgg gaggccgagg caggtggatc accctgaggt caggagtttg agactagcct     600
ggccaacatg aggaaaccct gtctactgaa aatacaaaaa ttagtcgggc gtggggaagg     660
gcgcctgtaa tcccaactac ttgggagact gaggcaggag aatcgtttga acctggaagg     720
cggaggttgc agtgagttga gacagcgtca ctgcactcca gcctgggcga cggagagaga     780
ctctgtttca aaaaacaaca acaaaaaaga aaactggcag gtcctacctg ccagtaagtg     840
gcagaaccca gacacaatgt gtttctccct tccccactct cagatctgta gactctaatc     900
ttggtgttct ttattctaca tggcatggga tctatgccac aaaaacattg atcttgctgc     960
cctgtagctg ttcacacatc ttcctcccct aagggtctga cagtctcttc ctcaaggaca    1020
gtgcctttgt ccaaccttat ctccagcctg cagggcctgg cacaggttct ggcttaaatg    1080
ctatactaac agtaattaca ttttcataaa atttatttta tgaaaactag gtactctcca    1140
aggtcctttg taccccatga gtctgagatt actaaactaa cagtttaaaa gattctggga    1200
```

accaggtgac cggggttctg gtcccaccag tatcctgtat tgtggctttg ggaaagtctg 1260
tttcccatct ctagacctca gtttccacac tttgaagagg aagtttgtgt ggtctctaag 1320
atccatttct gcgctctgta atctccataa gccaaggggt tcgaacctcc caggcttcac 1380
actgtgcagc tggccagcac cattcatcgc ccaccaacag ccctgggcat tggcctctga 1440
ttccccgtca cccagaggga agaccctttt ggggccgagg cgctcacctt cagccagtcc 1500
ccgtagtgga cgtagccccc gatgtaggcg gcgtaggtgc taatggccaa ttggagtgcg 1560
gcccccagcg cgaaccagcg ccgcttcacg ccaaaggaca tgaaactagc gcacagcacg 1620
gctgccccca tgtcgaaata caggtaaggc actgggatgt cgggcttcct gcgagccggg 1680
agtggggaag caacgaggac agaatgaagc ggtgggggaa gaagcttgag accccttgag 1740
ttcccacagg ctccactgct cccccaaacc agactcccct cccatgggca ggaccgccct 1800
accagatttg gtccgacacc cagaccagaa cccctcccag ggaggtccca attcctccca 1860
gttcaagggt cccgacttct agcccccaat ttcttgccca catcaccgcc ccctgccgcg 1920
tttagcggct gcgtaggcct gctcaccggc gtgcctcagc cctctcagcg tacagcatga 1980
gctggctgaa gcagccccaa aaggggcagc gtgtgagcag caccgaaccc aactgcatga 2040
tcagctgcaa catccaccgt ctcgaaccta tcttcgacgc catcttggga aagggcagtc 2100
cgctgcggcc tcaccccagt cagagaagca ggcgcgggga tgggacacgc agcttccggg 2160
tcaagcgctt tttctttttc ctcccggaaa caacggtgtc attctctagt tccggatcta 2220
aggtgaccca gttgcgaagt ccagccttct cgtctgctca tggagccttc agggccatat 2280
ttgtccctgc gaatttgaaa actagactag aggaaggaac agtgggttca ggatagcaga 2340
agtcttggac cgtagtatga tgtattgaag caaactggtc tttgaatgca gcccgatcag 2400
ggccctaatc ctgattccaa ccctgtgtga ttctgggcac atcacctaaa ctttatgaga 2460
ctcatcatga aatggggata attacaccta cctcagagtt gtgaactttt tttttctttt 2520
ttttttgttt tgttttgttt ttgagacgga gtctcactct gtcgcctagg ctggagtgca 2580
atggcacgat cttggctcac tgtaacctcc gcaacctggg ttcaagcgat tctcctgcct 2640
cagcctcctg agtagctgag actacaggct ctcgccacca cgcccggcta attttttgta 2700
tttttagtag agacggagtt tcaccgtctt agccaggatg gtctcgatct cctgacttcg 2760
tgatccgccc gcctcggcct cccaaagtgc tgggattaca ggcatgagcc accgcgccag 2820
gccagagttg tgaactttaa atgtgacaag gtcggtccta gtacatagta gtggttcaat 2880
aaatgataca cataacgcac cagaggaaac cccgtttct gttaagatca aagtaagggg 2940
tcctcctggc tcaaagtcac ctctttgggt ttgcctttg cctgtgccct gagagaaaaa 3000
ggagatctga acatctggga catctcaagt cagtccattt ctccagctgc catgctcccg 3060
ctgtaaccca aattgtcata ctttgtcagt ttaattgcag cagacatcca ggtggtctct 3120

gcattcccct ccagtgtgtt ctcagcccct tctcccacgc cccacccgtt tatatgttct 3180

cataacatca gctaccttcc tttgaatcac ttggcacggt ggcaattaga tagatagata 3240

gatatagata cacacgcaca cacacacaca cacacatata tatatatctg ctttacatat 3300

atatgctttt tttttatttt tgagacaggg tctcactgtg tcacccaggc tggagtgcag 3360

tggtgcgatc ttggctcact acagcctaca cctcccaggt tcaagcgatt ctcctgcctc 3420

agcttcccaa gtagctggga ttactggcgc ttgccaccac acccggctaa tttttatttt 3480

tagtggagac agggtttcac catgttggcc aggctgctct cgaactccta acctcaagcg 3540

atccaaccgc cttggcctcc caaagcgcta ggattacagg cgtgagacac cgcacccagc 3600

caatatattt gtgattgttt tattgacaat cttcccctac tagatcgcaa gctccctaag 3660

cacaaacacg gtgcctgggt ttctgtttac tgtttacaga tgctccttga cttgtgatgg 3720

agttatatcc tgataaaccc atcataggtt gaaaatactg taagttgaaa atgtgtggct 3780

gactggaagc tgtggcttgc tgacactgcc cagcattgta agagaaatac agtttccact 3840

gaatgcagat ctgctttcgc accaatgtaa agttgaaaac tccaatgctg ggaaccatct 3900

gtagtataca ggatgccctc aggacctagt atacaggatg ccctcaggaa atatgtgctg 3960

tacagataaa tgaaaaggtg ttgggctggg cgcggtggct aacgcctgtg atcccagcac 4020

tttaggaggc cgagtcgggt ggatcacgag gtcaggagtt caagaccagc ctggccaaga 4080

tggtgaaacc ccgtctctac taaaaataca aaaattagcc aga 4123

<210> 2
<211> 123
<212> DNA
<213> Artificial Sequence

<220>
<223> bisulfite-treated polynucleotides

<400> 2

aagggguagc gtgtgaguag uaucgaauuu aautguatga tuagutguaa uatuuaucgt 60

utcgaauuta tuttcgacgu uatuttggga aaggguagtu cgutgcgguu tuauuuuagt 120

uag 123

<210> 3
<211> 24
<212> DNA
<213> Artificial Sequence

<220>
<223> primer

<400> 3

aaggggtagc gtgtgagtag tatc 24

<210> 4
<211> 25
<212> DNA
<213> Artificial Sequence

<220>
<223> primer

<400> 4
ctaactaaaa taaaaccgca acgaa 25

<210> 5
<211> 33
<212> DNA
<213> Artificial Sequence

<220>
<223> primer

<400> 5
gggatattaa gtggagttat tttggtttta gtt 33

<210> 6
<211> 21
<212> DNA
<213> Artificial Sequence

<220>
<223> primer

<400> 6
ccctcccaac atccttccta a 21

<210> 7
<211> 123
<212> DNA
<213> Homo sapiens

<400> 7

```
aaggggcagc gtgtgagcag caccgaaccc aactgcatga tcagctgcaa catccaccgt    60
ctcgaaccta tcttcgacgc catcttggga aagggcagtc cgctgcggcc tcaccccagt   120
cag                                                                 123
```

## Description

### TECHNICAL FIELD

The present invention relates to a method for acquiring information on gynecologic cancer in a blood sample of a subject and the use of a gynecologic cancer detection kit for acquiring information on gynecologic cancer in a blood sample of a subject.

### BACKGROUND

Gynecologic cancer is a general term for malignant tumors arising in female-specific organs such as uterus, ovary and vagina. Among many types of gynecologic cancer, uterine cervix cancer, uterine body cancer and ovarian cancer particularly gain wide-spread prevalence. In the diagnosis of gynecologic cancer, an examination such as an internal examination, a cytological diagnosis and a tissue diagnosis is carried out depending on the area in which the development of cancer is suspected. However, these examinations are often painful and accompanied by hemorrhage due to the insertion of specialized tools, and therefore a significant burden is placed on patients.
In the examination of gynecologic cancer, the measurement of a tumor marker such as CA125 and CA19-9 is also carried out. However, the detection sensitivities of these tumor markers are often insufficient, and these tumor markers are also used for the detection of different types of cancer from gynecologic cancer.
In recent years, as a novel cancer diagnosis method, a method in which the diagnosis is carried out on the basis of information on genes has been studied. The method includes, for example, a method in which the diagnosis is carried out on the basis of information on the methylation of DNA.
In this method, a CpG site (5'-(CG)-3') contained in a nucleotide sequence for a certain gene is used. Information, such as the presence or absence of cancer cells, is acquired on the basis of the result of the analysis on the state of methylation of the marker, and the diagnosis of cancer is carried out employing the information as an indication.
de Souza JES et al. (S-Score: A Scoring System for the Identification and Prioritization of Predicted Cancer Genes, PLoS ONE, vol.9, e94147 (2014)) discloses a method in which a gene-specific score, i.e., a so-called S-score, is calculated on the basis of various types of information including the state of methylation of a gene and the expression amount of a gene, and then it is determined whether or not a gene of interest is a cancer gene or a tumor suppressor gene on the basis of the score.
In this document, Transmembrane protein 101 (TMEM101) gene is estimated as a tumor suppressor gene from the S-score of the gene in ovarian cancer.
Steinbach (2014 "Genomweite methylierungsanalysen und identifiziering prognostischer und prädiktiver marker fûr das epitheliale ovarialkarzinom") discloses that the promoter region of the TMEM101 gene is hyper-methylated in ovarian cancer. Steinbach does not make the comparison with other cancer types and hence, does not relate to a gynecologic cancer-specific diagnostic marker allowing the detection in other samples than tumour samples, e.g. blood samples.
Kolbe et al. (2012 PLOS One 7(3):e32941) studied the methylation status of 14495 genes in different types of gynaecologic cancer, but TMEM101 was not identified as being differently methylated as compared to normal samples.

### SUMMARY OF THE INVENTION

#### Problem to be solved by the Invention

There are an extremely small number of genes which can be used as markers for detecting gynecologic cancer. Therefore, the further development of a novel marker for detecting gynecologic cancer utilizing the analysis on methylation of a gene has been demanded.

#### Solution to the Problem

According to the present invention, a method for acquiring information on gynecologic cancer in a blood sample is provided. The method comprises the steps of analyzing methylation status of a CpG site in the promoter region of TMEM101 (Transmembrane protein 101) gene having the nucleotide sequence represented by SEQ ID NO:7 contained in a DNA sample prepared from the biological sample; and acquiring information that the blood sample comprises a gynecologic cancer cell when it is determined that the methylation occurs in the promoter region. In particular embodiments, the analyzing step is conducted by at least one method selected from the group consisting of mass spectrometry and a methylation-specific PCR method. In particular embodiments the gynecologic cancer is at least one type of cancer selected from the group consisting of ovarian cancer and uterine body cancer. In particular embodiments the analyzing step comprises: calculating methylation frequency of the promoter region, and the acquiring step comprises: comparing the frequency with a predetermined threshold; and acquiring information that the biological sample comprises the gynecologic cancer cell when the frequency is equal to or higher than the threshold.
In particular embodiments the method comprises the step of preparing a DNA sample from said biological sample. In further particular embodiments, the preparing step comprises treating DNA in a biological sample with bisulfite.

According to the present invention, the use of a reagent kit for the detection of gynecologic cancer in a blood sample of a subject is provided, said kit containing a primer that can specifically hybridize to a promoter region of TMEM101 gene, wherein the promoter region comprises: a uracil base converted from unmethylated cytosine base by a bisulfite treatment; and methylated cytosine base, and wherein the primer comprises the nucleotide sequence represented by SEQ ID NO: 3 or 4. In particular embodiments, the primer is used for analyzing methylation status of a CpG site in the promoter region of TMEM101 (Transmembrane protein 101) gene having the nucleotide sequence represented by SEQ ID NO:7 contained in the DNA sample.

Effect of the Invention

The present invention can provide new marker for detection of gynecologic cancer in a blood sample of a subject.

BRIEF DESCRIPTION OF THE DRAWINGS

Fig. 1 shows a graph illustrating methylation-positive fractions in a promoter region of TMEM101 gene, which are calculated from the methylation data on gynecologic cancer-affected tissues, gynecologic cancer-unaffected tissues and normal tissues.
Fig. 2 shows a graph illustrating methylation-positive fractions in the promoter region of TMEM101 gene, which are calculated from the methylation data on various clinical specimens.
Fig. 3 shows photographs illustrating the results of the amplification of DNA molecules, which are respectively extracted from normal tissues and cancer-affected tissues collected from gynecologic cancer patients, by a methylation-specific PCR (MSP) method.
Fig. 4 shows photographs illustrating the results of the amplification of DNA molecules, which are respectively extracted from various cancer tissues and normal tissues of various organs, by an MSP method.
Fig. 5 is a schematic view illustrating one example of a determination device for providing information on gynecologic cancer in a subject.
Fig. 6 is a block diagram illustrating the functional configuration of the determination device shown in Fig. 5.
Fig. 7 is a block diagram illustrating the hardware configuration of the determination device shown in Fig. 5.
Fig. 8 is a flow chart of the determination for the purpose of providing information on gynecologic cancer in a subject, using the determination device shown in Fig. 5.

DETAILED DESCRIPTION OF THE PREFERRED EMBODIMENTS

The methods described herein are carried out on a DNA sample prepared from a biological sample. In the method for acquiring information on gynecologic cancer (also simply referred to as the "method", herein below), firstly a DNA sample is prepared from a biological sample collected from a subject.

In the methods described herein, the type of the gynecologic cancer is not particularly limited, as long as the type is known as a type of gynecologic cancer in the art. Examples of the type of gynecologic cancer include uterine body cancer, ovarian cancer, uterine cervix cancer, fallopian tube cancer, vulvar cancer and vaginal cancer. Among these types of cancer, the method envisaged herein is particularly suitable for the acquisition of information on uterine body cancer and ovarian cancer. It is known that ovarian cancer is clinicopathologically classified into various tumors. In the methods provided herein, the type of ovarian cancer is not limited particularly, and is preferably a tumor arising in a surface epithelium, including clear cell adenocarcinoma, endometrioid adenocarcinoma and serous adenocarcinoma.

In the methods described herein, the biological sample is not particularly limited, as long as the biological sample is one containing DNA of a subject. The biological sample is preferably a sample containing genomic DNA, such as a clinical specimen. Examples of the clinical specimen include a body fluid, urine, cells collected in a cytologic examination and tissues collected by a surgery or a biopsy. Examples of the body fluid include blood, serum, plasma, lymph, ascitic fluid, bone marrow fluid and nipple discharge. A culture produced by culturing cells or tissues collected from a subject can also be used as the biological sample. A formalin-fixed paraffin-embedded (FFPE) sample of tissues collected from a subject can also be used as the biological sample.

The preparation of the DNA sample from the biological sample can be carried out by extracting DNA from the biological sample. The method for extracting DNA from the biological sample is known in the art. For example, DNA can be extracted by mixing the biological sample with a treatment solution containing a surfactant capable of lysing cells or tissues (e.g., sodium cholate, sodium dodecyl sulfate) and then subjecting the resultant mixed solution to a physical treatment (e.g., stirring, homogenizing, ultrasonic disruption) to release DNA contained in the biological sample into the mixed solution. In this case, it is preferred that the mixed solution is centrifuged to precipitate cell debris and the supernatant containing released DNA is used in the subsequent analysis step. The supernatant may be purified by a method known in the art. The extraction and purification of DNA from the biological sample may be carried out using a commercially available kit.

It is preferred that the preparation step additionally involves a step of fragmenting the extracted DNA. When the DNA is fragmented into proper lengths, it becomes possible to carry out the methylated DNA immunoprecipitation (MeDIP) method and the unmethylated cytosine conversion treatment mentioned below with high efficiency. The fragmentation of the DNA can be carried out by an ultrasonic treatment, a treatment with an alkali, a treatment with a restriction enzyme or the like. For example, when the fragmentation of the DNA is carried out by a treatment with an alkali, the DNA can be fragmented by adding a sodium hydroxide solution to a DNA solution at a final concentration of 0.1 to 1.0 N and then incubating the resultant solution at 10 to 40°C for 5 to 15 minutes. When the fragmentation of the DNA is carried out by a treatment with a restriction enzyme, the restriction enzyme to be used is selected as appropriate depending on the type of the nucleotide sequence for the DNA, and MseI, BamHI and the like can be used.

In the methods envisaged herein, the presence or absence of the methylation of a CpG site present in the promoter region of TMEM101 gene contained in the DNA sample is determined. The term "CpG site" as used herein refers to a site of a nucleotide sequence comprising a cytosine base (C) and a guanine base (G) arranged adjacently in this order in the direction from 5' to 3', wherein "p" in the CpG represents a phosphodiester bond between the cytosine base and the guanine base.

The nucleotide sequence for the promoter region of TMEM101 gene is known in the art, and can be known from a known database such as a database (http://www.ncbi.nlm.nih.gov/) which is provided by the National Center for Biotechnology Information (NCBI) of the United States National Library of Medicine.

ID numbers for TMEM101 gene are shown in Table 1. The nucleotide sequence (chr17:42090238-42094360) for the promoter region of TMEM101 gene is represented by SEQ ID NO: 1.

[Table 1]

| Symbol of gene | Unigene ID | Entrez Gene ID | SEQ ID NO: |
|---|---|---|---|
| *TMEM101* | Hs. 514211 | 84336 | 1 |

In the methods provided herein, the determination of the presence or absence of the methylation of a CpG site can be carried out on the basis of the result of the methylation analysis on the promoter region. The methylation analysis can be carried out by, for example, determining whether or not cytosine in at least one of CpG sites present in the promoter region of TMEM101 gene is methylated. In this case, the methylation analysis may be carried out in one CpG site. It is preferred to analyze on the presence or absence of methylation of multiple CpG sites.

Alternatively, the methylation analysis may be carried out by determining the frequency of methylation in the promoter region of TMEM101 gene. For example, the "frequency of methylation" may be the ratio of the number of methylated CpG sites to the number of CpG sites present in the promoter region. The analysis target region may be the whole area of the promoter region or a portion of the promoter region which contains at least one CpG site. The analysis target region may contain only one CpG site, and preferably contains multiple CpG sites. The position or positions and the number of a CpG site or CpG sites present in the promoter region of TMEM101 gene are known. Therefore, the number of methylated CpG sites in the promoter region can be employed as the frequency of methylation.

The frequency of methylation may be "a methylation score" which is obtained by analyzing the state of methylation of a CpG site in DNA by mass spectrometry such as below-mentioned MassARRAY (registered trademark). In MassARRAY (registered trademark), a DNA fragment is measured and a methylation score is calculated from the ratio of the area of a peak derived from a methylated DNA fragment to that of a peak derived from an unmethylated DNA fragment.

In the methods envisaged herein, the frequency of methylation in the promoter region of TMEM101 gene may be calculated manually or using a machine such as a computer.

In the methods envisaged herein, it is not particularly limited as to which CpG site (or a predetermined region containing the CpG site) in the promoter region of TMEM101 gene is to be analyzed, and the analysis target region can be selected as appropriate by a person skilled art. The position or positions and the number of a CpG site or CpG sites present in the promoter region of TMEM101 gene are known. Therefore, the CpG site or the range of the area to be analyzed can be determined by a routine experiment using a known analysis method as mentioned below.

As the means for analyzing the methylation, various analysis methods are known in the art. In the methods envisaged herein, it is not limited particularly as to what type of analysis method is to be employed, and a method is preferred which comprises a step of distinguishing methylated DNA from unmethylated DNA, a step of amplifying DNA and a step of detecting the methylated DNA and/or the unmethylated DNA.

As suitable methods for distinguishing methylated DNA from unmethylated DNA, a step of carrying out a treatment with a methylation-sensitive restriction enzyme, an MeDIP method, an unmethylated cytosine conversion treatment or the like can be mentioned. As the step of amplifying DNA, a step of carrying out a PCR method, a quantitative PCR method, an IVT (in vitro transcription) amplification method, an SPIA (trademark) amplification method or the like can be mentioned. As the step of detecting methylated DNA and/or unmethylated DNA, a step of carrying out an electrophoresis method, a sequence analysis method, a microarray analysis method, mass spectrometry, southern hybridization or the like can be mentioned.

An MeDIP method is a method comprising concentrating methylated DNA contained in a biological sample by immunoprecipitation using an anti-methylated cytosine antibody, an anti-methylated cytidine antibody or an antibody capable of recognizing a methylated DNA-binding protein specifically. It may be possible to concentrate methylated DNA contained in DNA obtained in the extraction step by an MeDIP method and carry out the methylation analysis on the methylated DNA. It may also be possible to amplify the methylated DNA, which has been concentrated by the MeDIP method, by an IVT amplification method or the like and carry out the methylation analysis on the amplification product using a microarray. This type of analysis method is called "an MeDIP on chip method".

An unmethylated cytosine conversion treatment is a treatment comprising reacting DNA extracted from a biological sample with an unmethylated cytosine converting agent to convert unmethylated cytosine in the DNA into another base (an uracil, thymine, adenine or guanine base). The unmethylated cytosine converting agent is a substance which can react with DNA to convert unmethylated cytosine contained in the DNA into another base (uracil, thymine, adenine or guanine). As the unmethylated cytosine converting agent, a bisulfite (hydrogen sulfite), such as sodium bisulfite, potassium bisulfite, calcium bisulfite and magnesium bisulfite, can be used suitably.

In the treatment of DNA with a bisulfite, unmethylated cytosine in the DNA is converted into uracil through a deamination reaction, while methylated cytosine does not undergo such base conversion. Therefore, the difference in the state of methylation of a CpG site (i.e., the difference between being methylated and unmethylated) in DNA results in the difference in a nucleotide sequence (i.e., the difference between containing C and containing U) when the CpG site is subjected to an unmethylated cytosine conversion treatment with a bisulfite. Such an unmethylated cytosine conversion treatment with a bisulfite is called "a bisulfite treatment".

When the bisulfite treatment is to be carried out, the amount (concentration) of the bisulfite to be added is not particularly limited, as long as unmethylated cytosine in DNA can be converted satisfactorily. The amount of the bisulfite to be added is for example, 1 M or more, preferably 1 to 15 M, more preferably 3 to 10 M, in terms of final concentration in a solution containing the DNA. The conditions (i.e., temperature and time) for incubation to be carried out after the addition of the bisulfite can be set as appropriate depending on the amount of the bisulfite added. For example, when the bisulfite is added at a final concentration of 6M, the incubation is carried out at 50 to 80°C for 10 to 90 minutes.

The methylation of a CpG site contained in DNA can be analyzed by analyzing the nucleotide sequence of the DNA that has been treated with the bisulfite and then detecting the difference between the nucleotide sequence and the original (i.e., untreated) one of the DNA. This method is called "a bisulfite sequencing method".

The methylation of a CpG site can also be analyzed by mass spectrometry. Specifically, PCR amplification is carried out using bisulfite-treated DNA as a template and using a primer set specific to a nucleotide sequence to be analyzed, and then the resultant PCR product is further subjected to IVT amplification to convert methylated cytosine and uracil to guanine (G) and adenine (A), respectively. The IVT amplification product is cleaved with RNase A, and then the difference in the mass (16 Da), which is resulted from the difference between G and A, between the obtained cleaved fragments can be detected on a MALDI-TOF (matrix assisted laser desorption/ionization time-of-flight) type mass spectrometer to analyze the state of methylation of the DNA. This method is called "MassARRAY (registered trademark) analysis".

It is known that the site cleaved with RNase A in the IVT product is a site located between an arbitrary base and the adjacent uracil (U) or thymine (T) base. Therefore, the nucleotide sequence and mass of the IVT product cleaved with RNase A can be predicted from the nucleotide sequence of the DNA that is used as the template (i.e., template DNA). Therefore, it is possible to determine as to from which site in the nucleotide sequence of the template DNA each of peaks obtained in MassARRAY (registered trademark) comes. For example, when one CpG site is methylated in a DNA fragment, a peak obtained in MassARRAY (registered trademark) shifts by 16 Da to the higher mass side. In the analysis of a DNA fragment having multiple CpG sites, for example, the DNA fragment shows a shift by 32 Da when two CpG sites are methylated in the DNA fragment, and the DNA fragment shows a shift by 48 Da when three CpG sites are methylated in the DNA fragment.

In mass spectrometry such as MassARRAY (registered trademark), the methylation score of the determined DNA fragments can be calculated. For example, when the ratio of the area of a peak for an unmethylated DNA fragment to the area of a peak for a methylated DNA fragment is 1 : 3 in a chart obtained by the analysis in a DNA fragment comprising a specific sequence, the methylation score of the DNA fragment is 75% (3/(1 + 3) = 0.75). The methylation score is theoretically 1 for a DNA fragment in which all of CpG sites are methylated, and is theoretically 0 for a DNA fragment in which all of CpG sites are not methylated.

The methylation of a CpG site can be analyzed by a methylation-specific PCR (MSP) method. An MSP method is a method in which the presence or absence of methylation of a CpG site is analyzed by carrying out PCR amplification of bisulfite-treated DNA using the below-mentioned primer and then confirming the presence or absence of a PCR product. In the MSP method, a primer set which enables the amplification of a nucleotide sequence in which a CpG site to be analyzed is methylated (i.e., cytosine is not converted to uracil) but does not enable the amplification of a nucleotide sequence in which a CpG site to be analyzed is not methylated (i.e., the cytosine is converted to uracil) is used.

In the MSP method using the primer set, it is determined that the CpG site to be analyzed is methylated when a PCR product is obtained. The MSP method can also be carried out using a primer set which does not enable the amplification of a nucleotide sequence in which cytosine in a CpG site to be analyzed is not converted to uracil but enables the amplification of a nucleotide sequence in which cytosine in a CpG site to be analyzed is converted to uracil. In this case, it is determined that the CpG site to be analyzed is methylated when no PCR product is obtained.

The presence or absence of the PCR product can be confirmed by, for example, a gel electrophoresis method. It is also possible to electrophorese a reaction solution obtained after the amplification on a gel and confirm the presence or absence of a band derived from the PCR product in the gel with naked eyes. Alternatively, it is also possible to obtain an image of the gel after the electrophoresis and confirm the presence or absence of the band by an image analysis. The image analysis can be carried out by, for example, obtaining a value representing the density of a pixel (e.g., a band intensity) in a region in which the presence of the PCR product is predicted in the image of the gel and then comparing the value with a predetermined threshold value. Specifically, it can be determined that the PCR product is obtained when the value representing the density of the pixel is equal to or more than the predetermined threshold value, while it can be determined that the PCR product is not obtained when the value representing the density of the pixel is less than the predetermined threshold value. The predetermined threshold value relating to the density of the pixel is not particularly limited, and may be a value representing the density of a pixel in background or may be a value two or three times greater than the value.

Each of the primers in the primer set used in the MSP method can be designed as appropriate by a person skilled in the art depending on the type of the nucleotide sequence containing a CpG site to be analyzed. It is preferred that the primer is designed in such a manner that cytosine in the CpG site to be analyzed is contained at the 3' terminal or in the vicinity thereof of the primer.

The methylation of a CpG site can also be analyzed with a microarray. In this case, the microarray to be used for the analysis can be prepared by immobilizing a nucleic acid probe complementary to the nucleotide sequence of the promoter region of TMEM101 gene onto a substrate. The microarray can be prepared by a method known in the art.

In the analysis with a microarray, it is preferred that DNA extracted from the biological sample is labelled with a labelling substance known in the art. Therefore, it is preferred that the determination method in this case additionally involves a step of labelling the extracted DNA. It is advantageous that the labelling step is carried out after the above-mentioned DNA amplifying step, because all of DNA molecules contained in the biological sample can be labeled by the labeling step. Examples of the labelling substance include a fluorescence substance, a hapten such as biotin and a radioactive substance.

Examples of the fluorescence substance include Cy3, Cy5, FITC and Alexa Fluor (trademark). The labeling of the DNA as mentioned above enables the easy measurement of a signal coming from the probe on the microarray. The method for labeling the DNA with the labeling substance is known in the art.

The signal may be a signal that is suitable for the type of the microarray. For example, the signal may be an electric signal that is generated when a DNA fragment hybridized to each of the probes on the microarray exists. In the case where the DNA to be analyzed is labelled in the above-mentioned manner, the signal may be a signal such as a fluorescence or luminescence emitted from the labelling substance. The detection of the signal can be carried out using a scanner provided in a conventional microarray analyzer. Examples of the scanner include GeneChip (registered trademark) Scanner3000 7G (Affymetrix) and Illumina (registered trademark) BeadArray Reader (Illumina).

In particular, when an analysis result that a methylated CpG site is present is obtained by the above-mentioned methylation analysis, it can be determined that methylation occurs in the CpG site present in the promoter region of TMEM101 gene. Alternatively, when a result that the methylation frequency obtained by the methylation analysis is higher than or equal to a predetermined threshold value is obtained, it can be determined that methylation occurs in the CpG site present in the promoter region of TMEM101 gene. The predetermined threshold value is not limited particularly, and can be set empirically from the accumulation of data on various biological samples. Alternatively, the predetermined threshold value may be set in the following manner. Firstly, the methylation frequency in DNA extracted from each of a biological sample which is confirmed in advance that cancer cells originated from gynecologic cancer are not contained (e.g., normal uterine body or ovarian tissues or cells) and a biological sample which contains cancer cells originated from gynecologic cancer is analyzed. Subsequently, on the basis of the analysis result, a threshold value is selected within a range which is higher than the methylation frequency in the biological sample that does not contains the cancer cells and is lower than the methylation frequency of the biological sample that contains the cancer cells. Preferably, a value which enables the highly accurate discrimination between the biological sample that does not contains the cancer cells and the biological sample that contains the cancer cells is selected as the threshold value.

In the methods envisaged herein, information on gynecologic cancer in a subject is acquired on the basis of the determination result obtained in the determination step. The information on gynecologic cancer refers to information on whether or not a biological sample collected from a subject contains cancer cells originated from gynecologic cancer. In particular, the information may be information that can serve as an indication for the diagnosis of gynecologic cancer or information for aiding the diagnosis of gynecologic cancer. The information is preferably information about the occurrence of gynecologic cancer in a subject or the state of gynecologic cancer in a subject, or both. For example, the information is information about the possibility of the occurrence of gynecologic cancer in a subject, information about the risk of occurrence of gynecologic cancer in a subject or the like. When gynecologic cancer is developed in a subject, the information on gynecologic cancer may be information about prognosis of gynecologic cancer in a subject, information about the degree of progression (stage) of the cancer in a subject or the like.

Obtaining information on gynecologic cancer in a subject can refer to the prediction, diagnosis, or prognosis of gynecologic cancer in said subject. In particular, when it is determined that a methylated CpG site is present in the determination step, information that the biological sample contains cancer cells originating from gynecologic cancer can be acquired. Alternatively, information suggesting that gynecologic cancer is developed or information that the state of gynecologic cancer is not good (or is bad) can also be acquired. Alternatively, information that the subject has a high risk of suffering from gynecologic cancer or information that the subject has been already affected by gynecologic cancer can also be acquired. When the subject has been already affected by gynecologic cancer, information that the prognosis in the subject is not good (or is bad) or information that the state of the cancer is in a further progressed stage can be acquired.

In contrast, when it is determined that no methylated CpG site is present in the determination step, information that the biological sample does not contain cancer cells originated from gynecologic cancer is acquired. Alternatively, information suggesting that gynecologic cancer is not developed or information that the state of gynecologic cancer is good can also be acquired. Alternatively, information that the subject has a low risk of suffering from gynecologic cancer or information that the subject is not affected by gynecologic cancer yet can also be acquired. When the subject has been already affected by gynecologic cancer, information that the prognosis in the subject is good or information that the state of the cancer is not in a progressed stage can be acquired.

The scope of the disclosure also includes a marker for use in the acquisition of information on gynecologic cancer by a methylation analysis (the marker is also simply referred to as "a marker", hereinbelow). The marker may be a nucleic acid which is isolated from a subject and contains at least one CpG site selected from CpG sites present in the promoter region of TMEM101 gene. The state of methylation in the marker can be analyzed, and information on gynecologic cancer in the subject can be acquired on the basis of the result of the analysis. The analysis on the state of methylation and the acquisition of the information on gynecologic cancer are as mentioned above.

In particular, as a marker for use in the acquisition of information on gynecologic cancer, a nucleic acid derived from a nucleic acid isolated from a subject can be used. The nucleic acid which is derived from a nucleic acid isolated from a subject and can be used as the marker comprises a nucleotide sequence produced by a bisulfite treatment of a nucleotide sequence for the whole area of a promoter region of TMEM101 gene or a contiguous nucleotide sequence which is a portion of the nucleotide sequence for the whole area of the promoter region of TMEM101 gene, wherein the promoter region contains a CpG site and a cytosine base that is not involved in the constitution of a CpG site. The cytosine that is not involved in the constitution of a CpG site may be any one which is different from cytosine contained in a CpG site, and is for example cytosine contained in a nucleotide sequence in which cytosine (C) and adenine (A), thymine (T) or cytosine (C) are arranged adjacently in this order in the direction from 5' to 3' (i.e., CA, CT or CC). The nucleic acid can be obtained by the bisulfite treatment of a nucleic acid isolated from a subject.

In the nucleic acid which can be used as the marker, when the isolated DNA is subjected to the bisulfite treatment, unmethylated cytosine in the isolated DNA is converted to uracil, while methylated cytosine remains unconverted. Information on gynecologic cancer can be acquired by analyzing the state of methylation of a CpG site in a polynucleotide. The isolated DNA can be prepared in the same manner as for the above-mentioned preparation of the DNA sample. The bisulfite treatment, the analysis on the state of methylation and the acquisition of information on gynecologic cancer can also be carried out in the same manner as mentioned above.

The size of the nucleic acid to be used as the marker is not particularly limited, as long as the methylation analysis of the nucleic acid can be carried out by an MSP method, a sequencing method or mass spectrometry. The size is preferably 50 to 200 nucleotides , more preferably 80 to 130 nucleotides. An example of the nucleic acid which can be used as the marker is a nucleic acid comprising the nucleotide sequence represented by SEQ ID NO: 2. The nucleic acid comprising the nucleotide sequence represented by SEQ ID NO: 2 is suitable for the analysis on the state of methylation by an MSP method.

The scope of the disclosure also includes a gynecologic cancer detection kit (which is also simply referred to as "a kit", hereinbelow). The kit may include a primer for amplifying a bisulfite-treated nucleic acid. The primer can hybridize specifically with a nucleic acid comprising a nucleotide sequence which is contained in the promoter region of TMEM101 gene and has a CpG site and in which cytosine present in a region other than the CpG site is converted to other base, and can also hybridize with a region containing a methylated CpG site. Accordingly, the primer may be capable of hybridizing specifically with nucleotide sequence comprising both a methylated CpG and a cytosine which is not in a CpG site and converted to another base. In particular, the first primer may hybridize specifically with a nucleotide sequence containing a methylated CpG site in the promoter region of TMEM101 gene.

The primer included in the kit may be a primer which can be used for an analysis method involving PCR amplification, such as an MSP method and a bisulfite sequencing method, or for the methylation analysis of a CpG site by mass spectrometry such as MassARRAY (registered trademark). Preferably, the primer is a primer which can be used for an MSP method or mass spectrometry such as MassARRAY (registered trademark). The nucleotide sequence of the primer can be set as appropriated depending on the type of the nucleotide sequence of the promoter region. For example, a primer comprising the nucleotide sequence represented by SEQ ID NO: 3 and a primer comprising the nucleotide sequence represented by SEQ ID NO: 4 can be mentioned.

The kit may additionally contain a second primer. The second primer can hybridize with a nucleic acid comprising a nucleotide sequence which is contained in the promoter region of TMEM101 gene and contains a CpG site and in which cytosine in the CpG site is converted to another base, and can also hybridize with a region containing a sequence in which cytosine in an unmethylated CpG site is converted to another base through a conversion treatment.

The present disclosure also includes a program product by which a computer can execute the acquisition of information on gynecologic cancer in a subject. Examples of the program product include a program which can be downloaded through an internet or the like and a recording medium on which the program is recorded and which can be read out with a computer.

For example, a program for allowing a computer to execute the following steps is exemplified:

a step of acquiring a result of an analysis on the methylation of a CpG site present in the promoter region of TMEM101 gene contained in a DNA sample collected from a subject from a measurement device; and

a step of determining the presence or absence of gynecologic cancer cells in the DNA sample on the basis of the acquired analysis result.

Hereinbelow, an exemplary device suitable for achieving the method described herein will be described with reference to drawings. Fig. 5 is a schematic view illustrating one example of a diagnosis aid device which can be used for acquiring information on gynecologic cancer in a subject. The diagnosis aid device 10 illustrated in Fig. 5 is equipped with a measurement device 20 and a determination device 30 connected to the measurement device 20.

In this example, when the methylation analysis is carried out by an MSP method, the measurement device 20 may be a gel imaging derive such as a fluorescent image scanner. In this case, a reaction solution obtained by nucleic acid amplification by the MSP method is subjected to gel electrophoresis, and then a gel after the electrophoresis is set on the measurement device 20 to detect an amplification product by the measurement device 20. The measurement device 20 acquires gel image information of the amplification product and provides the acquired information to the determination device 30.

The measurement device 20 may be an MALDI-TOF type mass spectrometer. In this case, the measurement device 20 acquires mass spectrometric information of a substance of interest, such as a flight time or a mass-to-charge ratio (an m/z value). When a measurement sample prepared from a DNA sample from the subject is set on the measurement device 20, the measurement device 20 acquires mass spectrometric information of a nucleic acid contained in the measurement sample and provides the acquired mass spectrometric information to the determination device 30.

The determination device 30 is equipped with a computer main body 300, an input unit 301 which is composed of a keyboard and a mouse, and a display unit 302 which is composed of an LCD and a CRT and can display sample information, determination results and the like. The determination device 30 acquires gel image information of the amplification product from the measurement device 20. On the basis of the information, the determination device 30 executes a program for providing information on gynecologic cancer in the subject. The determination device 30 may be a different device from the measurement device 20 as illustrated in Fig. 5, or may be a device accommodating the measurement device 20 therein. In the latter case, the determination device 30 may serve as the diagnosis aid device 10 by itself.

Fig. 6 is a block diagram illustrating a software configuration for the computer main body 300 in the determination device 30 with functional blocks. As illustrated in Fig. 6, the computer is equipped with an acquisition unit 321, a storage unit 322, a calculation unit 323, a determination unit 324 and an output unit 325. The acquisition unit 321 is connected to the measurement device 20 in a communicable manner through a network.

The acquisition unit 321 acquires the information provided by the measurement device 20. The storage unit 322 stores a formula for acquiring a threshold value or a band intensity both required for the determination, a processing program and the like. The calculation unit 323 calculates a band intensity in accordance with the stored processing program using the information acquired in the acquisition unit 321. The determination unit 324 determines whether or not the band intensity which is acquired in the acquisition unit 321 or is calculated in the calculation unit 323 is equal to or greater than the threshold value stored in the storage unit 322. The output unit 325 outputs the determination result obtained in the determination unit 324 to the display unit 302 as information on gynecologic cancer in the subject (e.g., the presence or absence of gynecologic cancer cells in the biological sample collected from the subject).

Fig. 7 is a block diagram illustrating the hardware configuration of the computer main body 300 illustrated in Fig. 6. As illustrated in Fig. 7, the computer main body 300 is equipped with a CPU (Central Processing Unit) 310, a ROM (Read Only Memory) 311, a RAM (Random Access Memory) 312, a hard disk 313, an input/output interface 314, a readout device 315, a communication interface 316 and an image output interface 317. The CPU 310, the ROM 311, the RAM 312, the hard disk 313, the input/output interface 314, the readout device 315, the communication interface 316 and the image output interface 317 are connected one another in a data-communicable manner through a bus 318.

The CPU 310 can execute a program stored in the ROM 311 and a program loaded into the RAM 312. By the execution of the programs by the CPU 310, each of the functions illustrated in Fig. 6 can be executed. In this manner, the determination device 30 functions as a determination device for providing the information on gynecologic cancer in the subject.

The ROM 311 is composed of a mask ROM, a PROM, an EPROM, an EEPROM and the like. In the ROM 311, a program to be executed by the CPU 310 in the above-mentioned manner and data to be used for the execution of the program are recorded.

The RAM 312 is composed of an SRAM, a DRAM and the like. The RAM 312 can be used for the read out of the programs recorded in the ROM 311 and the hard disk 313. The RAM 312 can also be used as a working area for the CPU 310 during the execution of these programs.

Into the hard disk 313, an operating system, a program such as an application program (e.g., a computer program for providing the information on gynecologic cancer in the subject) to be executed by the CPU 310 and data to be used for the execution of the program are installed.

The readout device 315 is composed of a flexible disk drive, a CD-ROM drive, a DVD-ROM drive and the like. The readout device 315 can read out a program or data recorded in a portable recording medium 40.

The input/output interface 314 is composed of a serial interface such as USB, IEEE1394 and RS-232C, a parallel interface such as SCSI, IDE and IEEE1284, and an analogue interface composed of a D/A converter, an A/D converter and the like. To the input/output interface 314, an input unit 301 such as a keyboard and a mouse is connected. An operator can input various commands to the computer main body 300 by means of the input unit 301.

The communication interface 316 is, for example, Ethernet (registered trademark) interface. The computer main body 300 can also transmit printing data to a printer or the like through the communication interface 316.

The image output interface 317 is connected to the display unit 302 composed of an LCD, a CRT and the like. Through this connection, the display unit 302 can output a video signal corresponding to the image data coming from the CPU 310. The display unit 302 displays an image (screen) in accordance with the input video signal.

Next, an exemplary processing procedure for acquiring the information on gynecologic cancer in the subject by the diagnosis aid device 10 will be described. Fig. 8 illustrates one example of the flow chart for acquiring information on gynecologic cancer. Here, a case in which a band intensity is obtained from gel image information acquired using a biological sample collected from a subject and it is determined whether or not the obtained band intensity is equal to or greater than a threshold value is described as an example.

Firstly, in step S1-1, the acquisition unit 321 in the diagnosis aid device 10 acquires gel image information on TMEM101 gene from the measurement device 20. Subsequently, in step S1-2, the calculation unit 323 acquires a band intensity from the gel image information and transmits the band intensity to the storage unit 322.

Subsequently, in step S1-3, the determination unit 324 carries out the determination whether or not the band intensity acquired in step S1-2 is equal to or greater than a threshold value stored in the storage unit 322. When the band intensity is equal to or greater than the threshold value, the routine proceeds to step S1-4 and the determination unit 324 transmits a determination result that gynecologic cancer cells are present in the biological sample collected from the subject to the output unit 325. On the other hand, when the band intensity is smaller than the threshold value, the routine proceeds to step S1-5 and the determination unit 324 transmits a determination result that no gynecologic cancer cell is present in the biological sample collected from the subject to the output unit 325.

Finally, in step S1-6, the output unit 325 outputs the determination result as the information on gynecologic cancer in the subject and allows the display unit 302 to display the information. In this manner, the diagnosis aid device 10 can provide information that aids the diagnosis on whether or not a subject is suffering from gynecologic cancer to physicians and the like.

Hereinbelow, the present invention will be described in detail by way of examples. However, the invention is not intended to be limited by these specific examples.

EXAMPLES

Example 1: Identification of novel markers utilizing data on methylation in gynecologic cancer-affected tissues, gynecologic cancer-unaffected tissues and normal mammary gland tissues

(1) Acquisition of methylation data

In Example 1, methylation data obtained using Infinium HumanMethylation450 BeadChip (Illumina), which are publicly available in TCGA (The Cancer Genome Atlas: http://tcga-data.nci.nih.gov/tcga/tcgaHome2.jsp), with respect to ovarian cancer-affected tissues (49 samples) and an ovarian cancer-unaffected tissue (i.e., an ovarian tissue) (1 sample), uterine body cancer-affected tissues (334 samples) and uterine body cancer-unaffected tissues (i.e., uterine body tissues) (36 samples) were acquired. Methylation data obtained using Infinium HumanMethylation450 BeadChip, which are publicly available in a document by Nazor KL. et al. (Recurrent variations in DNA methylation in human pluripotent stem cells and their differentiated derivatives. Cell Stem Cell 2012; 10(5) : 620 - 634), were also acquired with respect to normal tissues (23 samples).

(2) Identification of novel markers

Data mining using Infinium HumanMethylation450 BeadChip (Illumina) was carried out. As a result, promoter regions of TMEM101 gene were identified as markers which were methylated specifically in ovarian cancer- and uterine body cancer-affected tissues (see Fig. 1). The markers are referred to as "markers of this example", hereinbelow.

Example 2: Comparison of methylation data among cancer/tumor-affected tissue samples originated from multiple types of cancers/tumors, cancer-unaffected tissue samples and normal tissue samples

(1) Acquisition of methylation data

In Example 2, methylation data were compared among 15 types of cancer/tumor-affected tissue samples, 12 types of cancer-unaffected tissue samples and 19 types of normal tissue samples. The number of samples for each of the tissues is shown below.

[Table 2]

| Tissues | Number of samples |
|---|---|
| Brain tumor | 114 |
| Lung cancer | 370 |
| Breast cancer | 548 |
| Gastric cancer | 69 |
| Colorectal cancer | 324 |
| Liver cancer | 81 |
| Renal cancer | 363 |
| Bladder cancer | 78 |
| Uterine body cancer | 334 |
| Ovarian cancer | 49 |
| Prostate cancer | 153 |
| Acute leukemia | 192 |
| Sarcoma | 73 |
| Malignant melanoma | 242 |
| Thyroid cancer | 316 |

[Table 3]

| Tissues | Number of samples |
|---|---|
| Brain tumor | 1 |
| Lung cancer | 75 |
| Breast cancer | 98 |
| Gastric cancer | 2 |
| Colorectal cancer | 40 |
| Liver cancer | 14 |
| Renal cancer | 208 |
| Bladder cancer | 15 |
| Uterine body cancer | 36 |
| Ovarian cancer | 1 |
| Prostate cancer | 49 |
| Thyroid cancer | 36 |

[Table 4]

| Tissues | RCAST | Document 1 | Document 2 | Total |
|---|---|---|---|---|
| Normal brain (Brain) | 2 | 1 | 0 | 3 |
| Normal oral cavity (Oral) | 2 | 0 | 0 | 2 |
| Normal lung (Lung) | 0 | 2 | 0 | 2 |
| Normal colorectal mucosa (Colon) | 2 | 0 | 0 | 2 |
| Normal liver (Liver) | 2 | 0 | 0 | 2 |
| Peripheral blood from normal person (Blood) | 2 | 2 | 0 | 4 |
| Normal skeletal muscle (Skeletal) | 2 | 2 | 0 | 4 |
| Normal testis (Testis) | 1 | 0 | 0 | 1 |
| Normal gastric mucosa (Stomach) | 0 | 1 | 0 | 1 |
| Normal pancreas (Pancreas) | 0 | 2 | 0 | 2 |
| Normal spleen (Spleen) | 0 | 2 | 0 | 2 |
| Normal kidney (Kidney) | 0 | 0 | 0 | 0 |
| Normal adrenal gland (Adrenal grand) | 0 | 2 | 0 | 2 |
| Normal ureter (Ureter) | 0 | 2 | 0 | 2 |
| Normal bladder (Bladder) | 0 | 2 | 0 | 2 |
| Normal lymph nodes (Lymph nodes) | 0 | 2 | 0 | 2 |
| Normal adipose tissue (Adipose tissue) | 0 | 2 | 0 | 2 |
| Normal heart (Heart) | 0 | 1 | 0 | 1 |
| Normal various blood cell components (WB, PBMC, Gran, CD4+, CD8+, CD14+, CD19+, CD56+, Neu, Eos) | 0 | 0 | 60 | 60 |

In Table 4, with regard to the samples shown in the column "RCAST", methylation data were acquired by the present inventors by carrying out Infinium Methylation Assay using Infinium HumanMethylation450 BeadChip (Illumina).

With regard to the samples shown in the columns "Document 1" and "Document 2", methylation data obtained using Infinium HumanMethylation450 BeadChip (Illumina), which are publicly available in documents shown below, respectively, were acquired.

Document 1: Nazor KL et al., Recurrent variations in DNA methylation in human pluripotent stem cells and their differentiated derivatives. Cell Stem Cell 2012; 10(5): 620 - 634

Document 2: Reinius LE et al., Differential DNA Methylation in Purified Human Blood Cells: Implications for Cell Lineage and Studies on Disease Susceptibility, PLoS One, 7(7) e41361

The term "methylation data" as used herein refers to methylation ratios (mCpG) of CpG regions of TMEM101, which were acquired in the following manner. In Infinium HumanMethylation450 BeadChip, both a probe for methylation and a probe for non-methylation are provided in each of 482,421 CpG sites among all of CpG sites present on human genome. A signal intensity (signal M) of a probe for methylation of a CpG site in a gene of interest and a signal intensity (signal U) of a probe for non-methylation of the CpG site in the gene of interest were detected with BeadArray Reader, and the methylation ratio in the CpG region in the gene of interest was calculated in accordance with the following calculation formula.

$$(\text{mCpG}) = (\text{signal M})/[(\text{signal M}) + (\text{signal U})]$$

(2) Comparison of methylation-positive fractions among cancer/tumor-affected tissue samples, cancer-unaffected tissue samples and normal tissue samples

When a statistically significant difference was observed between the methylation ratio (mCpG) in a tumor-affected tissue sample and the methylation ratio (mCpG) in a normal tissue sample, the tumor-affected tissue sample was determined as being methylation-positive. The methylation-positive ratio (%) with respect to each of the cancer types was calculated.

$$\text{Methylation-positive fraction (\%)} = [(\text{number of methylation-positive samples})/(\text{total number of samples})] \times 100$$

(In the case of brain tumor for example, the methylation-positive fraction was calculated in accordance with the formula: [(number of methylation-positive samples in brain tumor-affected samples)/(total number of brain tumor-affected samples = 114)] × 100.)

The results are shown in Fig. 2. In Fig. 2, "normal tissues" refer to normal tissues shown in Table 4 excluding 60 samples of various normal blood cell components, and "normal blood cells" refer to 60 samples of various normal blood cell components. As shown in Fig. 2, the occurrence of the methylation in each of the markers of this example was rarely observed in the cancer-unaffected tissues, as well as the human normal tissues and the human normal blood cells. It was found that all of the markers of this example were highly methylated particularly in ovarian cancer and uterine body cancer compared with other types of cancer. Therefore, it was demonstrated that the markers of this example were particularly suitable for the detection of ovarian cancer and uterine body cancer among various types of gynecologic cancer.

Example 3: Comparison of methylation data between tissues from normal persons and tissues from gynecologic cancer patients

(1) Biological samples

In Example 3, as biological samples from gynecologic cancer patients, cancer-affected tissues collected from ovarian cancer patients (9 samples) and cancer-affected tissues collected from uterine body cancer patients (4 samples) were used. The ovarian cancer samples include clear cell adenocarcinoma samples (3 samples), endometrioid adenocarcinoma samples (3 samples) and serous adenocarcinoma samples (3 samples). As control samples, a normal ovary tissue (1 sample) and normal uterine body tissues (2 samples) were used.

(2) Preparation of measurement samples

(i) Extraction of genomic DNA

Genomic DNA was extracted from each of the tissue samples using QIAamp DNA Mini Kit (QIAGEN). As a control genomic DNA, genomic DNA of a human peripheral blood lymphocyte was used. The genomic DNA of the human peripheral blood lymphocyte was amplified using GenomiPhi v2DNA amplification kit (GE Healthcare Life Sciences). The resultant amplification product was composed of unmethylated DNA.

Subsequently, the amplification product was fragmented using Bioruptor (COSMO BIO) to produce a solution of unmethylated DNA fragments (0%-methylated DNA). A portion of the unmethylated DNA fragment solution was taken, and was then reacted with SssI methylase (New England Biolab) to methylate all of cytosine bases in CG sequences, thereby producing a solution of methylated DNA fragments (100%-methylated DNA).

(ii) Bisulfite treatment

Each (500 ng) of the DNA fragments produced in the above-mentioned procedure was subjected to a bisulfite treatment using EZ DNA Methylation Kit (Zymo Research), and the genomic DNA obtained after the treatment was eluted into sterilized distilled water (80 μl).

(3) Methylation-specific PCR (MSP)

MSP was carried out using the measurement samples and the control samples which were obtained in the above-mentioned step (2). The composition of a PCR reagent used, the primer sets used and the conditions for the PCR are shown below.

<PCR reagent>

DW (distilled water): 18.8 μL
10 × PCR Buffer with $MgCl_2$ (Roche): 2.5 μL
10 mM dNTP mix: 0.5 μL
10 μM sense primer: 1.0 μL
10 μM antisense primer: 1.0 μL
Faststart Taq polymerase (Roche): 0.2 μL
Measurement sample: 1.0 μL
Total: 25.0 μL

<Primer set>

A primer set used in the MSP is shown in Table 5. The primer set is one whereby it becomes possible to obtain an amplification product when a DNA region to be amplified is methylated (the primer set is also referred to as "a primer set for methylation detection", hereinbelow). As a primer set for accuracy management, a primer set for determining whether or not the bisulfite treatment was achieved properly was also used (see Table 6). The nucleotide sequence for a region to be analyzed with the primer set for methylation detection in the promoter region of TMEM101 gene is represented by SEQ ID NO: 7. The nucleotide sequence of the region that has been subjected to bisulfite conversion is represented by SEQ ID NO: 2.

[Table 5]

| Symbol of gene | Nucleotide sequence | SEQ ID NO: | PCR PRODUCT (bp) | Region to be amplified |
|---|---|---|---|---|
| *TMEM101* | AAGGGGTAGCGTGTGAGTAGTATC | 3 | 123 | chr17:42, 092, 238-42, 092, 360 |
| | CTAACTAAAATAAAACCGCAACGAA | 4 | | |

[Table 6]

| Primers for accuracy management | Nucleotide sequence | SEQ ID NO: | PCR PRODUCT (bp) |
|---|---|---|---|
| Forward | GGGATATTAAGTGGAGTTATTTGGTTTTAGTT | 5 | 129 |
| Reverse | CCCTCCCAACATCCTTCCTAA | 6 | |

<Conditions for PCR reaction>

6 minutes at 95°C,

X cycles of 30 seconds at 95°C, 15 seconds at 60°C and 30 seconds at 72°C,

7 minutes at 72°C, and

allowing to leave at 16°C.

The number of cycles "X" was 38 for the primer set for methylation detection and was 40 for the primer set for accuracy management.

(4) Results of analysis

The amplification products obtained in the above-mentioned MSP were confirmed by 2% agarose gel electrophoresis. The results are shown in Fig. 3. In this figure, the numbers of "0" and "100" in the controls represent "a 0%-methylated control sample" and "a 100%-methylated control sample", respectively.

As illustrated in Fig. 3, in the PCR using the primer set for accuracy management, a band was detected for all of the samples. The results demonstrate that the bisulfite treatment of all of the samples was achieved properly. In the PCR using the primer set for methylation detection, no band derived from a methylated CpG site was detected for each of the normal ovary tissue samples and the normal uterine body tissue samples. In contrast, a band was detected for 4 among the 9 ovarian cancer tissue samples. A band was detected for all of the 4 uterine body cancer tissue samples. Thus, in the methylation analysis on the markers of this example by the MSP method, it was suggested that the methylation of the markers of this example and gynecologic cancer were correlated with each other, as in the results obtained by Infinium method in Example 1. In other words, it was demonstrated that, TMEM101 served as a marker having such a high specificity that TMEM101 was methylated at high methylation ratios in ovarian cancer and uterine body cancer but could not be detected in normal ovary tissues and normal uterine body tissues.

Example 4: Comparison of methylation data in various cancer tissues and normal tissues

(1) Biological samples

In this example, as biological samples, tissue samples collected from patients suffering from various types of cancer and normal tissues of various organs were used. The details of each of the samples are shown in Table 7. In Table 7, "T" represents "a tumor tissue", "N" represents "a normal tissue", and " PBL" represents "a peripheral blood leukocyte".

[Table 7]

| Derived tissues | | Number of samples | Provider | Notation |
|---|---|---|---|---|
| Large intestine | Cancer | 2 | Purchased from Bio Chain Institute, Inc. | T1 |
| | | | | T2 |
| | Normal | 3 | | N1 |
| | | | | N2 |
| | | | | N3 |
| Brain | Cancer | 1 | Tokyo University | T |
| | Normal | 2 | Purchased from Bio Chain Institute, Inc. | N1 |
| | | | | N2 |
| Lung | Cancer | 1 | Purchased from Bio Chain Institute, Inc. | T |
| | Normal | 2 | Tokyo University | N1 |
| | | | | N2 |
| Mammary gland | Cancer | 1 | Purchased from Bio Chain Institute, Inc. | T |
| | Normal | 2 | | N1 |
| | | | | N2 |
| Liver | Cancer | 1 | Purchased from Bio Chain Institute, Inc. | T |
| | Normal | 2 | Tokyo University | N1 |
| | | | | N2 |
| Kidney | Cancer | 1 | Purchased from Bio Chain Institute, Inc. | T |
| | Normal | 2 | Tokyo University | N1 |
| | | | | N2 |
| Bladder | Cancer | 1 | Purchased from Bio Chain Institute, Inc. | T |
| | Normal | 1 | | N |
| Uterine cervix | Normal | 1 | Purchased from Bio Chain Institute, Inc. | Cervix |
| Testis | Normal | 1 | Purchased from Bio Chain Institute, Inc. | Testis |
| Blood cell | Normal | 1 | Purchased from Bio Chain Institute, Inc. | PBL |
| Stomach | Cancer | 1 | Purchased from Bio Chain Institute, Inc. | T |
| | Normal | 1 | | N |

(2) Preparation of measurement samples and control samples

(i) Extraction of genomic DNA

Genomic DNA was extracted from each of the tissue samples in the same manner as in Example 3. As control genomic DNA, genomic DNA of a human peripheral blood lymphocyte was used. A solution of unmethylated DNA fragments (0%-methylated DNA) and a solution of methylated DNA fragments (100%-methylated DNA) were prepared from the genomic DNA of the human peripheral blood lymphocyte in the same manner as in Example 3.

(ii) Bisulfite treatment

Each (500 ng) of the obtained DNA fragments was subjected to a bisulfite treatment using EZ DNA Methylation Kit (Zymo Research), and the treated genomic DNA was eluted into sterilized distilled water (80 μl).

(3) MSP

MSP was carried out using the measurement samples and the control samples (bisulfite-treated DNA molecules) obtained in the above-mentioned section (2). The primer sets, the composition of a PCR reagent and the reaction conditions for PCR employed in MSP in Example 4 were the same as those employed in Example 3.

(4) Analysis of results

The amplification products produced in the above-mentioned MSP were confirmed by 2% agarose gel electrophoresis. The results are shown in Fig. 4. In the figure, "NC" and "PC" represent "a 0%-methylated control sample" and "a 100%-methylated control sample", respectively. The "accuracy management" refers to an amplification product produced using the primer for accuracy management.

As illustrated in Fig. 4, in the PCR using the primer set for accuracy management, a band was detected for all of the samples. Therefore, it is found that the bisulfite treatment of the samples was achieved properly. In the PCR using the primer set for methylation detection, a band derived from a methylated CpG site was not detected in any one of the cancer tissue samples and the normal tissue samples. The results suggest that the markers of this example are gynecologic cancer-specific markers.

## Claims

**1.** A method for acquiring information on gynecologic cancer in a biological sample of a subject, comprising the steps of analyzing methylation status of a CpG site in the promoter region of TMEM101 (Transmembrane protein 101) gene having the nucleotide sequence represented by SEQ ID NO:7 contained in the DNA sample prepared from said biological sample; and
acquiring information that the biological sample comprises a gynecologic cancer cell when it is determined that the methylation occurs in the promoter region,
wherein said biological sample is a blood sample.

**2.** The method according to claim 1, wherein the analyzing step is conducted by at least one method selected from the group consisting of mass spectrometry and a methylation-specific PCR method.

**3.** The method according toclaim 1 or 2, wherein the gynecologic cancer is at least one type of cancer selected from the group consisting of ovarian cancer and uterine body cancer.

**4.** The method according to any one of claims 1 to 3, wherein
the analyzing step comprises: calculating methylation frequency of the promoter region, and
the acquiring step comprises: comparing the frequency with a predetermined threshold; and acquiring information that the biological sample comprises the gynecologic cancer cell when the frequency is equal to or higher than the threshold.

**5.** The method according to any one of claims 1 to 4, wherein the method comprises the step of preparing a DNA sample from said biological sample.

**6.** The method of claim 5, wherein said preparing step comprises treating DNA in a biological sample with bisulfite.

**7.** Use of a reagent kit for the detection of gynecologic cancer in a blood sample, said kit containing a primer comprising the nucleotide sequence represented by SEQ ID NO: 3 or 4,
wherein the primer is capable of specifically hybridizing to a promoter region of TMEM101 (Transmembrane protein 101) gene, wherein the promoter region comprises: a uracil base converted from unmethylated cytosine base by a bisulfite treatment; and methylated cytosine base.

**8.** Use according to claim 7 , wherein the primer is used for analyzing methylation status a CpG site in the promoter region of TMEM101 (Transmembrane protein 101) gene having the nucleotide sequence represented by SEQ ID NO:7, contained in the DNA sample.

**Patentansprüche**

**1.** Verfahren zum Erfassen von Informationen über gynäkologischen Krebs in einer biologischen Probe eines Individuums, umfassend die Schritte
Analysieren des Methylierungszustands einer CpG-Stelle in einer Promotorregion des TMEM101-(Transmembranprotein 101)-Gens mit der durch SEQ ID NO: 7 dargestellten Nukleotidsequenz, die in der aus der biologischen Probe präparierten DNA-Probe enthalten ist; und
Erfassen von Informationen, dass die biologische Probe eine gynäkologische Krebszelle umfasst, wenn bestimmt wird, dass die Methylierung in der Promotorregion auftritt, wobei die biologische Probe eine Blutprobe ist.

**2.** Verfahren nach Anspruch 1, wobei der Analyseschritt durch mindestens ein Verfahren durchgeführt wird, das aus der aus Massenspektrometrie und einem methylierungsspezifischen PCR-Verfahren bestehenden Gruppe ausgewählt ist.

**3.** Verfahren nach Anspruch 1 oder 2, wobei der gynäkologische Krebs mindestens eine Art von Krebs ist, ausgewählt aus der Gruppe, bestehend aus Eierstockkrebs und Gebärmutterkörperkrebs.

**4.** Verfahren nach einem der Ansprüche 1 bis 3, wobei
der Analyseschritt umfasst: Berechnen der Methylierungshäufigkeit der Promotorregion, und
der Erfassungsschritt umfasst: Vergleichen der Häufigkeit mit einem vorbestimmten Schwellenwert; und Erfassen von Informationen, dass die biologische Probe die gynäkologische Krebszelle umfasst, wenn die Häufigkeit gleich oder höher als der Schwellenwert ist.

**5.** Verfahren nach einem der Ansprüche 1 bis 4, wobei das Verfahren den Schritt Präparieren einer DNA-Probe aus der biologischen Probe umfasst.

**6.** Verfahren nach Anspruch 5, wobei der Präparierschritt das Behandeln von DNA in einer biologischen Probe mit Bisulfit umfasst.

**7.** Verwendung eines Reagenzkits zum Nachweis von gynäkologischem Krebs in einer Blutprobe, wobei der Kit einen Primer enthält, der die durch SEQ ID NO: 3 oder 4 dargestellte Nukleotidsequenz umfasst,
wobei der Primer spezifisch an eine Promotorregion des TMEM101-(Transmembranprotein 101)-Gens hybridisieren kann, wobei die Promotorregion umfasst: eine Uracilbase, die durch eine Bisulfitbehandlung aus unmethylierter Cytosinbase umgewandelt wird; und methylierte Cytosinbase.

**8.** Verwendung nach Anspruch 7, wobei der Primer zur Analyse des Methylierungszustands einer CpG-Stelle in der Promotorregion des in der DNA-Probe enthaltenen TMEM101-(Transmembranprotein 101)-Gens mit der durch SEQ ID NO: 7 dargestellten Nukleotidsequenz verwendet wird.

## Revendications

**1.** Procédé d'acquisition d'informations sur un cancer gynécologique dans un échantillon biologique d'un sujet, comprenant les étapes de
analyse d'état de méthylation d'un site CpG dans la région de promoteur du gène TMEM101 (protéine transmembranaire 101) ayant la séquence nucléotidique représentée par SEQ ID NO: 7 contenu dans l'échantillon d'ADN préparé à partir dudit échantillon biologique ; et
acquisition de l'information selon laquelle l'échantillon biologique comprend une cellule de cancer gynécologique lorsqu'il est déterminé que la méthylation se produit dans la région de promoteur, dans lequel ledit échantillon biologique est un échantillon de sang.

**2.** Procédé selon la revendication 1, dans lequel l'étape d'analyse est conduite par au moins un procédé choisi dans le groupe constitué de la spectrométrie de masse et un procédé de PCR spécifique de la méthylation.

**3.** Procédé selon la revendication 1 ou 2, dans lequel le cancer gynécologique est au moins un type de cancer choisi dans le groupe constitué d'un cancer de l'ovaire et d'un cancer du corps de l'utérus.

**4.** Procédé selon l'une quelconque des revendications 1 à 3, dans lequel
l'étape d'analyse comprend : le calcul de la fréquence de méthylation de la région de promoteur, et
l'étape d'acquisition comprend : la comparaison de la fréquence à un seuil prédéterminé ; et l'acquisition de l'information selon laquelle l'échantillon biologique comprend la cellule de cancer gynécologique lorsque la fréquence est égale ou supérieure au seuil.

**5.** Procédé selon l'une quelconque des revendications 1 à 4, le procédé comprenant l'étape de préparation d'un échantillon d'ADN à partir dudit échantillon biologique.

**6.** Procédé selon la revendication 5, dans lequel ladite étape de préparation comprend le traitement de l'ADN dans un échantillon biologique avec du bisulfite.

**7.** Utilisation d'une trousse de réactifs pour la détection d'un cancer gynécologique dans un échantillon de sang, ladite trousse contenant une amorce comprenant la séquence nucléotidique représentée par SEQ ID NO: 3 ou 4,
dans laquelle l'amorce est capable de s'hybrider spécifiquement avec une région de promoteur du gène de TMEM101 (protéine transmembranaire 101), dans laquelle la région de promoteur comprend : une base uracile convertie à partir de base cytosine non méthylée par un traitement par bisulfite ; et une base cytosine méthylée.

**8.** Utilisation selon la revendication 7, dans laquelle l'amorce est utilisée pour l'analyse d'état de méthylation d'un site CpG dans la région de promoteur du gène TMEM101 (protéine transmembranaire 101) ayant la séquence nucléotidique représentée par SEQ ID NO: 7, contenu dans l'échantillon d'ADN.

FIG. 1

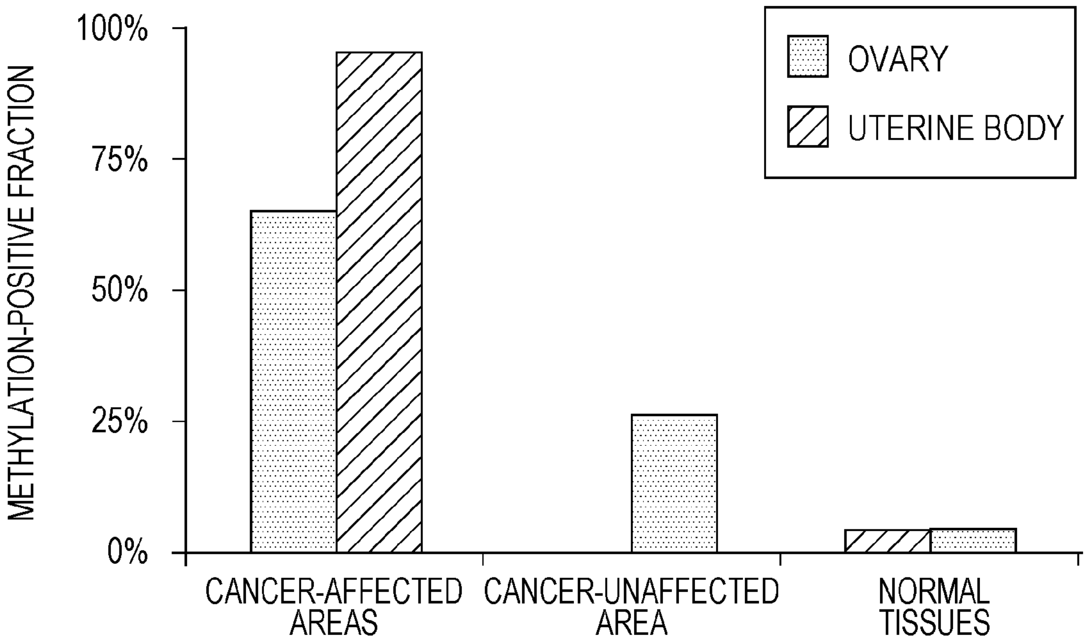
100%
75%
50%
25%
0%
METHYLATION-POSITIVE FRACTION
OVARY
UTERINE BODY
CANCER-AFFECTED AREAS
CANCER-UNAFFECTED AREA
NORMAL TISSUES

FIG. 2

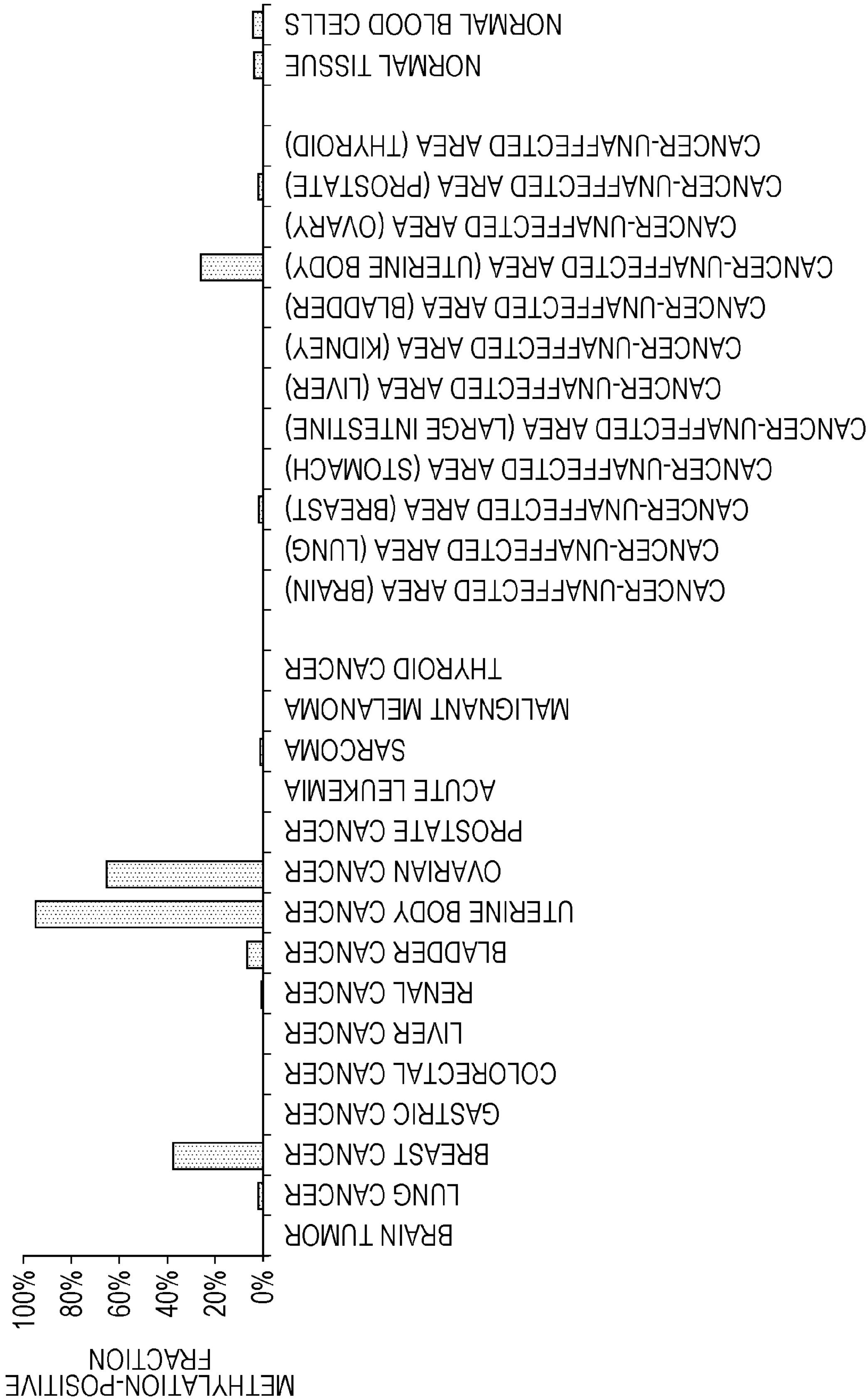
METHYLATION-POSITIVE FRACTION
100%
80%
60%
40%
20%
0%
BRAIN TUMOR
LUNG CANCER
BREAST CANCER
GASTRIC CANCER
COLORECTAL CANCER
LIVER CANCER
RENAL CANCER
BLADDER CANCER
UTERINE BODY CANCER
OVARIAN CANCER
PROSTATE CANCER
ACUTE LEUKEMIA
SARCOMA
MALIGNANT MELANOMA
THYROID CANCER
CANCER-UNAFFECTED AREA (BRAIN)
CANCER-UNAFFECTED AREA (LUNG)
CANCER-UNAFFECTED AREA (BREAST)
CANCER-UNAFFECTED AREA (STOMACH)
CANCER-UNAFFECTED AREA (LARGE INTESTINE)
CANCER-UNAFFECTED AREA (LIVER)
CANCER-UNAFFECTED AREA (KIDNEY)
CANCER-UNAFFECTED AREA (BLADDER)
CANCER-UNAFFECTED AREA (UTERINE BODY)
CANCER-UNAFFECTED AREA (OVARY)
CANCER-UNAFFECTED AREA (PROSTATE)
CANCER-UNAFFECTED AREA (THYROID)
NORMAL TISSUE
NORMAL BLOOD CELLS

FIG. 3

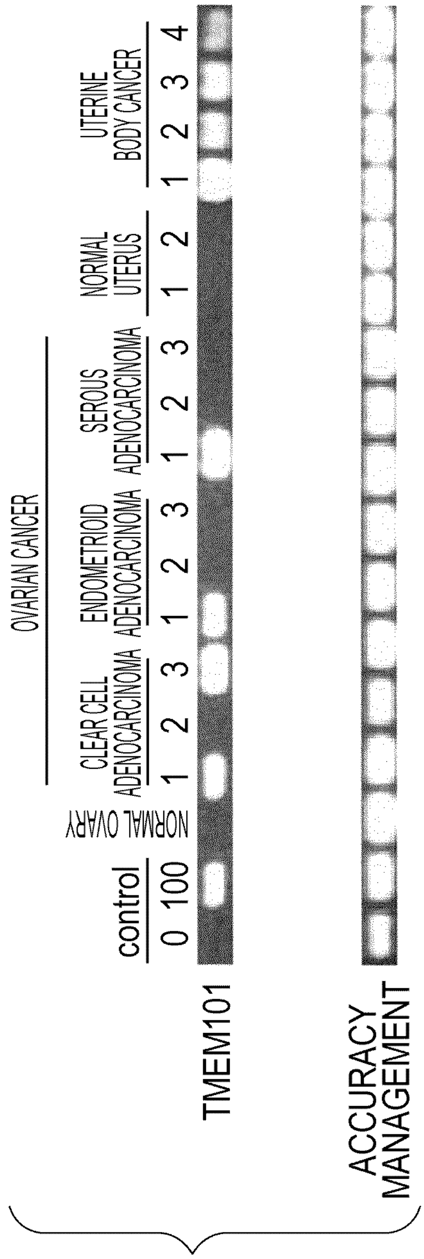
control
0 100
NORMAL OVARY
OVARIAN CANCER
CLEAR CELL ADENOCARCINOMA
1 2 3
ENDOMETRIOID ADENOCARCINOMA
1 2 3
SEROUS ADENOCARCINOMA
1 2 3
NORMAL UTERUS
1 2
UTERINE BODY CANCER
1 2 3 4
TMEM101
ACCURACY MANAGEMENT

FIG. 4

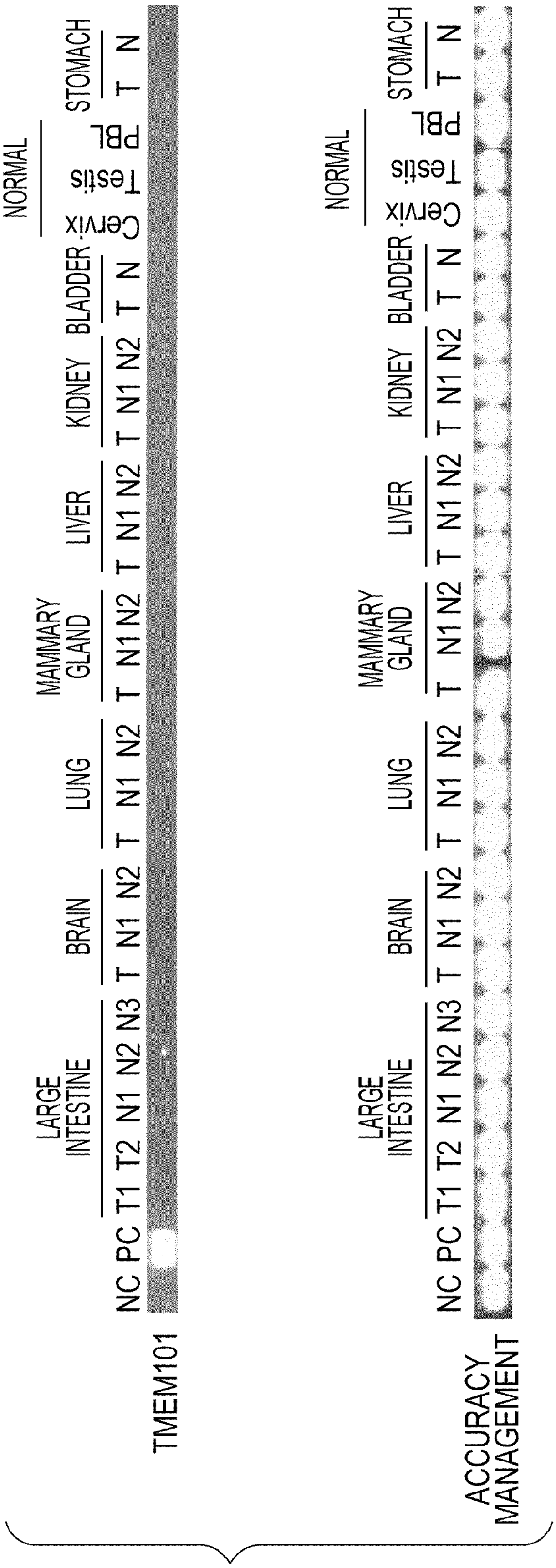
TMEM101
ACCURACY MANAGEMENT
NC PC
LARGE INTESTINE
T1 T2 N1 N2 N3
BRAIN
T N1 N2
LUNG
T N1 N2
MAMMARY GLAND
T N1 N2
LIVER
T N1 N2
KIDNEY
T N1 N2
BLADDER
T N
NORMAL
Cervix Testis PBL
STOMACH
T N

FIG. 5

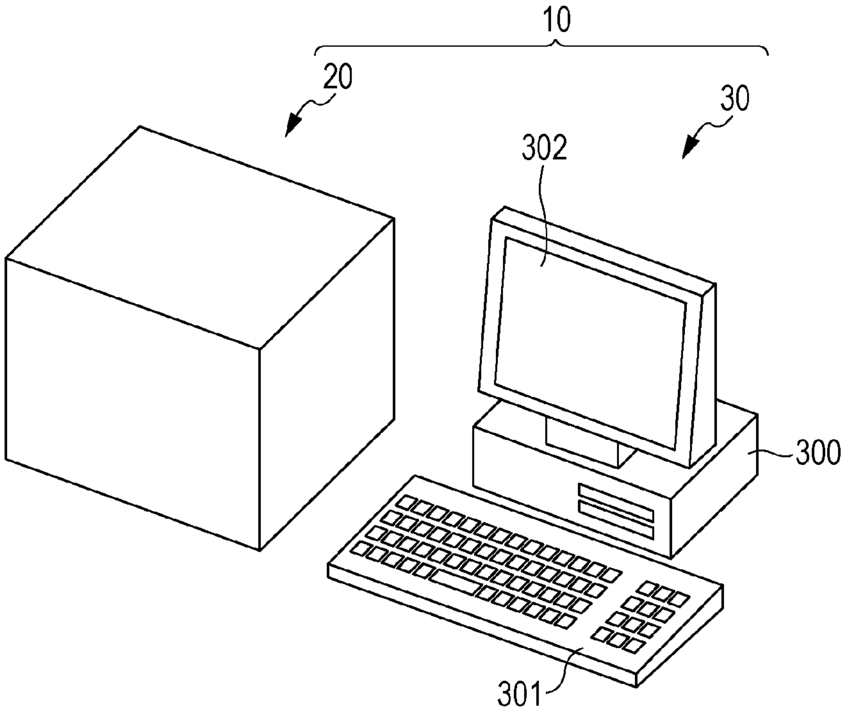
10
20
30
302
300
301

FIG. 6

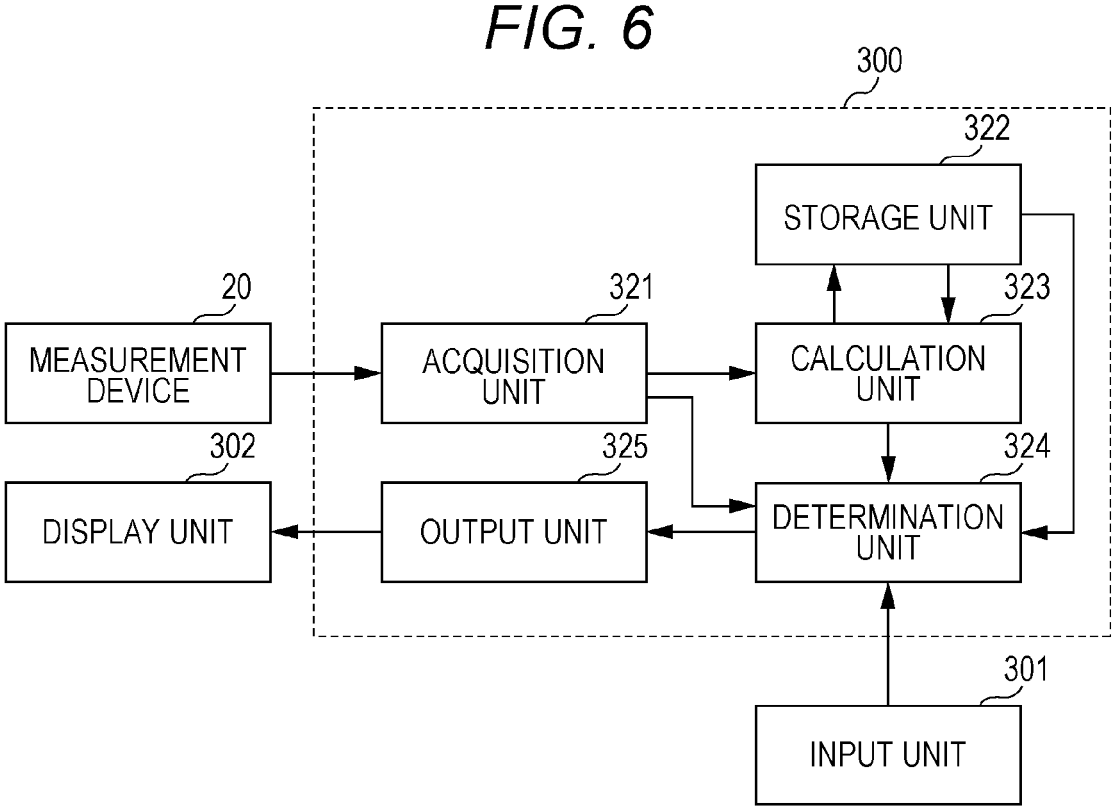
300
322
STORAGE UNIT
20
321
323
MEASUREMENT DEVICE
ACQUISITION UNIT
CALCULATION UNIT
302
325
324
DISPLAY UNIT
OUTPUT UNIT
DETERMINATION UNIT
301
INPUT UNIT

FIG. 7

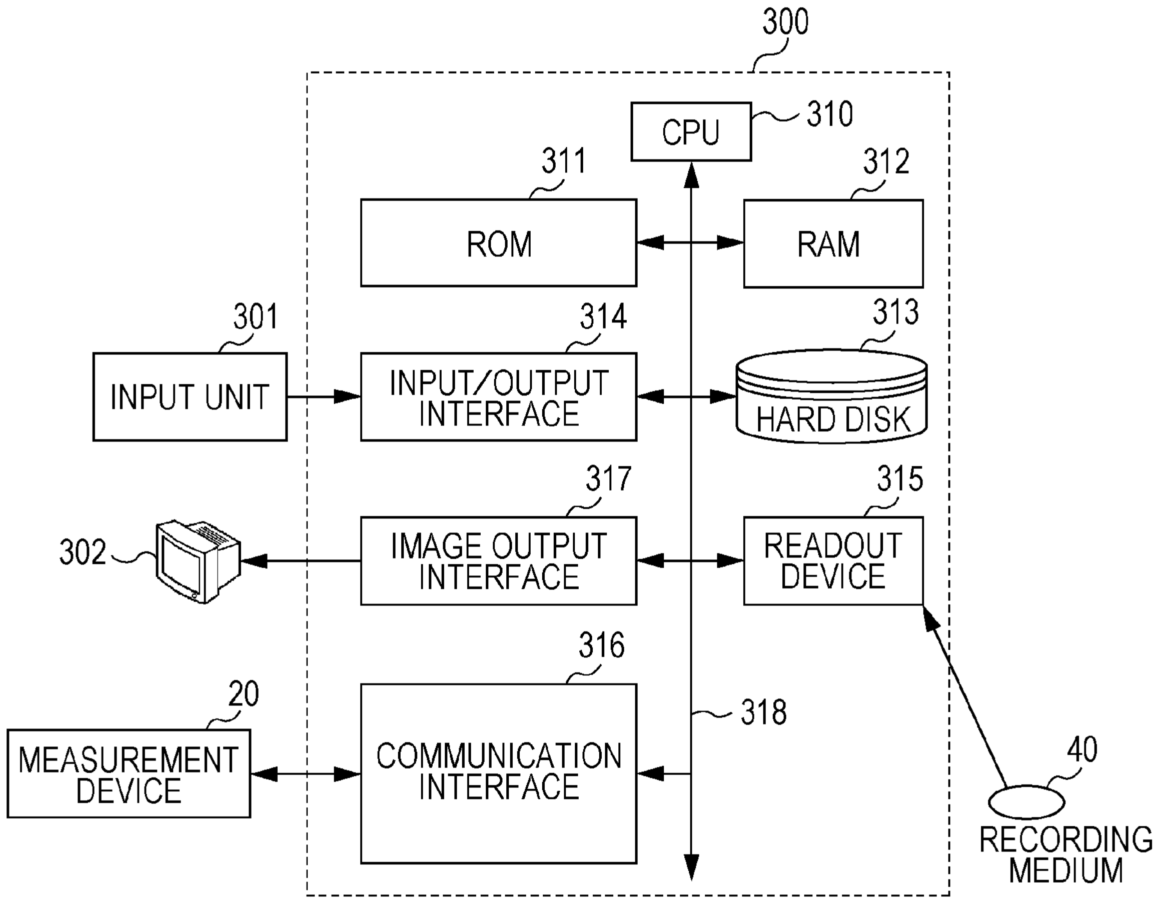
300
CPU
310
311
ROM
312
RAM
301
INPUT UNIT
314
INPUT/OUTPUT INTERFACE
313
HARD DISK
317
IMAGE OUTPUT INTERFACE
315
READOUT DEVICE
302
316
COMMUNICATION INTERFACE
20
MEASUREMENT DEVICE
318
40
RECORDING MEDIUM

FIG. 8

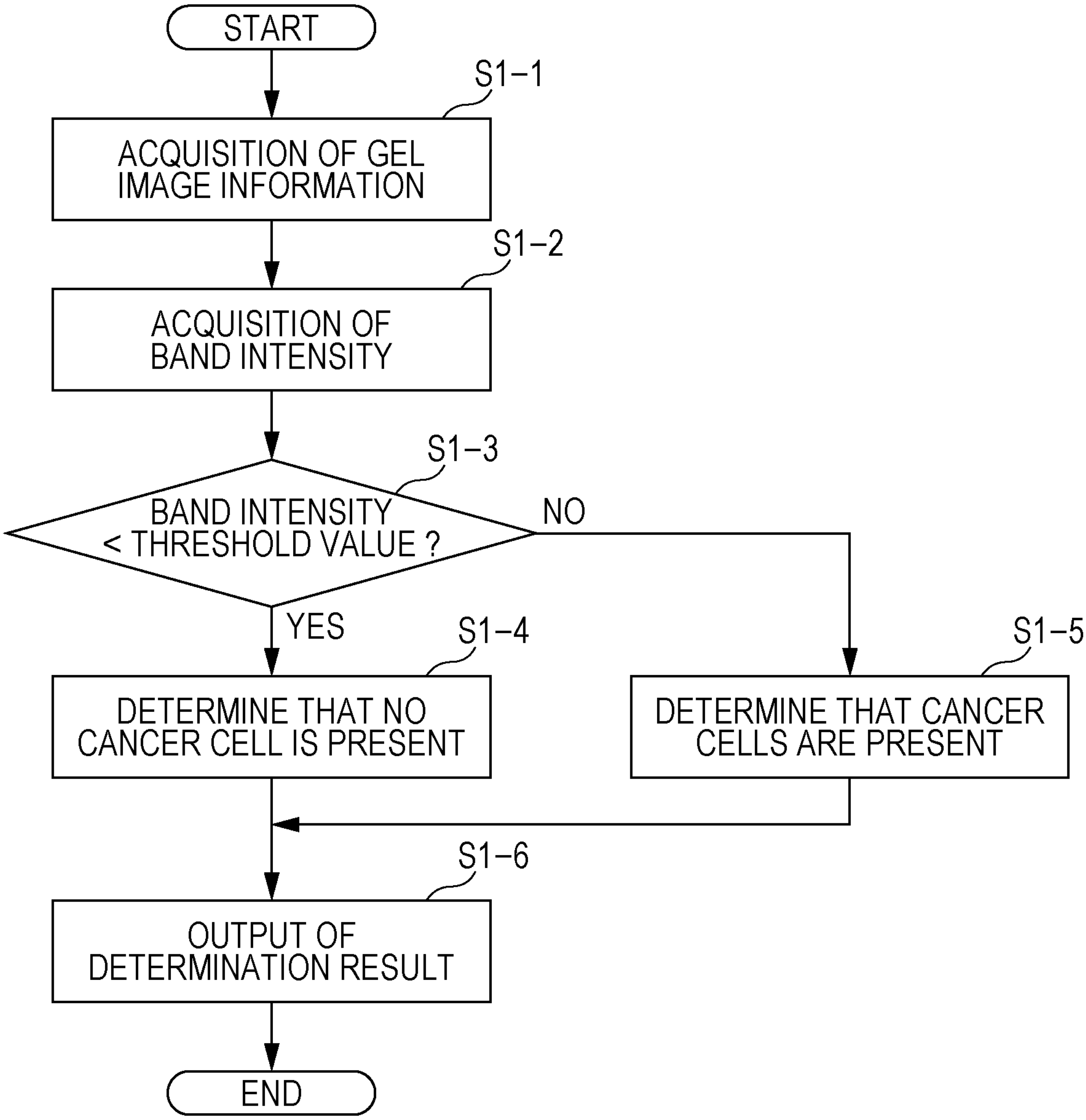
START
S1-1
ACQUISITION OF GEL IMAGE INFORMATION
S1-2
ACQUISITION OF BAND INTENSITY
S1-3
BAND INTENSITY < THRESHOLD VALUE ?
NO
YES
S1-4
DETERMINE THAT NO CANCER CELL IS PRESENT
S1-5
DETERMINE THAT CANCER CELLS ARE PRESENT
S1-6
OUTPUT OF DETERMINATION RESULT
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015073592 A

### Non-patent literature cited in the description


- **DE SOUZA JES et al.** S-Score: A Scoring System for the Identification and Prioritization of Predicted Cancer Genes. *PLoS ONE,* 2014, vol. 9, e94147
- **STEINBACH.** *Genomweite methylierungsanalysen und identifiziering prognostischer und prädiktiver marker fûr das epitheliale ovarialkarzinom,* 2014
- **KOLBE et al.** *PLOS One,* 2012, vol. 7 (3), e32941
- **NAZOR KL. et al.** Recurrent variations in DNA methylation in human pluripotent stem cells and their differentiated derivatives. *Cell Stem Cell,* 2012, vol. 10 (5), 620-634
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), e41361